(19) Europäisches Patentamt
European Patent Office
Office européen des brevets

(11) Veröffentlichungsnummer: **0 011 794**
**B1**

(12) # EUROPÄISCHE PATENTSCHRIFT

(45) Veröffentlichungstag der Patentschrift: **14.10.81**

(51) Int. Cl.³: **C 07 C 143/86**

(21) Anmeldenummer: **79104566.9**

(22) Anmeldetag: **19.11.79**

(54) **Verfahren zur Herstellung von Sulfamidsäurehalogeniden.**

(30) Priorität: **02.12.78 DE 2852274**

(43) Veröffentlichungstag der Anmeldung: **11.06.80 Patentblatt 80/12**

(45) Bekanntmachung des Hinweises auf die Patenterteilung: **14.10.81 Patentblatt 81/41**

(84) Benannte Vertragsstaaten: **BE CH DE FR GB IT NL**

(56) Entgegenhaltungen: **DE-A-2 514 937**

(73) Patentinhaber: **BASF Aktiengesellschaft, Carl-Bosch-Strasse 38, D-6700 Ludwigshafen (DE)**

(72) Erfinder: **Jacobs, Peter, Dr. Dipl.-Chem., Hanns-Fay-Strasse 4, D-6710 Frankenthal (DE)**
Erfinder: **Hamprecht, Gerhard, Dr. Dipl.-Chem., Rote-Turm-Strasse 28, D-6940 Weinheim (DE)**
Erfinder: **Mangold, Dietrich, Dr. Dipl.-Chem., Hermann-Walker-Strasse 49, D-6903 Neckargemuend (DE)**

**EP 0 011 794 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung von Sulfamidsäurehalogeniden

Die Erfindung betrifft ein neues Verfahren zur Herstellung von Sulfamidsäurehalogeniden durch Umsetzung von Sulfamidsäuren mit Halogen in Gegenwart von Phosphor oder Phosphortrihalogenid, wobei die Sulfamidsäuren zuerst aus Isocyanaten oder Harnstoffen hergestellt und einbadig dann in vorgenannter Weise umgesetzt werden können.

Es ist aus der deutschen Offenlegungsschrift 21 64 176 bekannt, daß man Sulfamidsäurehalogenide durch Umsetzung von Sulfamidsäuren mit einem Säurehalogenid der schwefligen Säure, Phosphorsäure, phosphorigen Säure, Kohlensäure oder Oxalsäure herstellt. Thionylchlorid, Thionylbromid, Phosphorpentachlorid, Phosphortrichlorid, Phosphoroxychlorid, Phosphorpentabromid, Phosphortribromid, Phosgen, Oxalsäurechlorid und Oxalsäurebromid werden als bevorzugte Säurehalogenide offenbart. Wie die Ausführungsbeispiele zeigen , wird jeweils nur ein einziges Säurehalogenid verwendet. Außer Phosgen und Phosphorpentachlorid in je einem Beispiel wird nur Thionylchlorid als Ausgangshalogenid in den übrigen Beispielen veranschaulicht. Das Verfahren ist mit Bezug auf Umweltschutz im Falle der Verwendung von Thionylhalogeniden und mit Bezug auf Ausbeute und Reinheit des Endstoffs im Falle der übrigen Säurehalogenide, z. B. Phosphorpentachlorid (Beispiel 2b), unbefriedigend. Bei der Synthese werden unter Verwendung von Thionylchlorid pro Mol Sulfamidsäurehalogenid neben 1 Mol Chlorwasserstoff stets 1 Mol Schwefeldioxid gebildet, daß im Abgasstrom abgetrennt, mit Natronlauge als Bisulfid aufgefangen, konzentriert und deponiert werden muß. Weiterhin gestaltet sich die Abtrennung des bei der Reaktion verwendeten überschüssigen Thionylchlorids von den vorzugsweise als Lösungsmitteln verwendeten chlorierten Kohlenwasserstoffen aufgrund der meist geringen Siedepunktdifferenz schwierig. Führt man die Umsetzung mit Phosphortrichlorid durch, das lediglich erwähnt, aber in den Ausführungsbeispielen nicht verwendet wird, so erhält man unbefriedigende Ergebnisse.

Die deutsche Offenlegungsschrift 25 14 937 beschreibt eine weitere, auch in Bezug auf den Umweltschutz vorteilhaftere Synthese von Sulfamidsäurehalogeniden aus den entsprechenden Sulfamidsäuren, wobei man die Umsetzung mit Phosphorpentahalogenid als Säurehalogenid in einer Menge von 0,35 bis 0,6 Mol, bezogen auf 1 Mol Ausgangsstoff, in Gegenwart von 1 bis 5 Mol Phosphoroxyhalogenid, bezogen auf 1 Mol Phosphorpentahalogenid, und in Gegenwart von 50 bis 300 Gewichtsprozent Halogenkohlenwasserstoff als Lösungsmittel, bezogen auf Ausgangsstoff, durchführt. Sie erwähnt auch die in der US-Patentschrift 19 06 440 beschriebene Möglichkeit, zunächst aus Phosphortrichlorid oder Phosphor in Phosphoroxychlorid mit Chlor Phosphorpentachlorid herzustellen, weist aber ausdrücklich daraufhin, daß zuerst diese Herstellung des Phosphorpentachlorid erfolgen muß und danach mit der so erhaltenen Suspension von Phosphorpentachlorid die Sulfamidsäuren umgesetzt werden. Beispiel 4 zeigt, daß die so gebildeten Suspensionen vor Zugabe der Sulfamidsäure das aus den Ausgangsstoffen gebildete Phosphorpentachlorid, in Phosphoroxychlorid suspendiert, enthalten. Auch wird das in der US-Patentschrift 1 906 440 gezeigte Verfahren dort ausdrücklich als eine Methode zur Herstellung von Suspensionen von feinverteiltem Phosphorpentachlorid beschrieben. Die US-Patentschrift zeigt in Beispiel 3 anhand einer Umsetzung mit p-Nitrobenzoesäure, daß nur eine solche Suspension von Phosphorpentachlorid für spätere Synthesen in Betracht kommt. Nachteilig bei diesem Verfahren sind die Verwendung des schwer zugänglichen, unwirtschaftlichen Phosphorpentachlorids bzw. die umständliche und zusätzliche Herstellungsoperation dieses Chlorids.

Es wurde nun gefunden, daß man Sulfamidsäurehalogenide der Formel

$$\begin{array}{l} R—N—SO_2X \\ \quad\;\, | \\ \quad\;\, H \end{array} \qquad (I)$$

in der R einen aliphatischen oder cycloaliphatischen Rest bedeutet und X ein Halogenatom bezeichnet, durch Umsetzung von Sulfamidsäuren oder ihren sulfamidsauren Metallsalzen mit einem Halogenierungsmittel in Gegenwart eines Lösungsmittels vorteilhaft erhält, wenn man

a) eine Sulfamidsäure der Formel

$$\begin{array}{l} R—N—SO_3H \\ \quad\;\, | \\ \quad\;\, H \end{array} \qquad (II)$$

in der R die vorgenannte Bedeutung hat, oder ihr sulfamidsaures Metallsalz mit Halogen als Halogenierungsmittel in Gegenwart von Phosphor oder Phosphortrihalogenid umsetzt, oder

b) in einer ersten Stufe eine Isocyanat der Formel

$$R—N=C=O \qquad (III)$$

in der R die vorgenannte Bedeutung hat, mit Schwefelsäure zu einer Sulfamidsäure der Formel

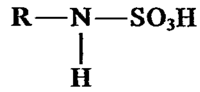
(II)

in der R die vorgenannte Bedeutung hat, und dann in einer zweiten Stufe den Stoff II oder sein sulfamidsaures Metallsalz mit Halogen als Halogenierungsmittel in Gegenwart von Phosphor oder Phosphortrihalogenid umsetzt, oder

c) einen substituierten Harnstoff der Formel

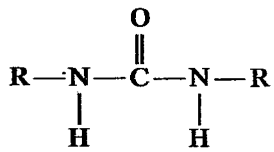
(IV)

worin R die vorgenannte Bedeutung hat, mit Oleum, das Schwefeltrioxid in einer Menge von 1 bis 2,5 Mol und Schwefelsäure in einer Menge von 1 bis 1,5 Mol je Mol Ausgangsstoff II enthält, in einem ersten Schritt bei einer Temperatur von −20 bis +50°C und dann in einem zweiten Schritt bei einer Temperatur zwischen 50°C und 140°C umsetzt, und dann die erhaltene Sulfamidsäure der Formel

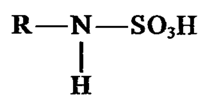
(II)

in der R die vorgenannte Bedeutung hat, oder ihr sulfamidsaures Metallsalz in einem dritten Schritt mit Halogen als Halogenierungsmittel in Gegenwart von Phosphor oder Phosphortrihalogenid umsetzt.

Die Umsetzung läßt sich für den Fall der Verwendung von Äthylsulfamidsäure, Phosphor bzw. Phosphortrihalogenid und Chlor durch folgende Formeln wiedergegeben:

$$2\ C_2H_5\text{—}NHSO_3H + 2\ P + 5\ Cl_2 \xrightarrow{-2\ POCl_3} 2\ C_2H_5\text{—}NHSO_2Cl + 2\ HCl$$

$$C_2H_5NH\text{—}SO_3H + PCl_3 + Cl_2 \xrightarrow{-POCl_3} H_5C_2\text{—}NH\text{—}SO_2Cl + HCl$$

Im Vergleich zu dem bekannten Verfahren unter Verwendung von schwefelfreien Halogeniden liefert das Verfahren nach der Erfindung auf einfacherem und wirtschaftlicherem Wege Sulfamidsäurehalogenide in guter Ausbeute und guter Reinheit. Im Vergleich zu Umsetzungen mit Thionylhalogeniden ist Ausbeute und Reinheit ebenfalls gut oder besser, der wesentliche Vorteil des erfindungsgemäßen Verfahrens liegt aber in der leichteren Aufarbeitung, geringeren Abgas- und Abwasserschwierigkeiten und somit in besserem Umweltschutz. Gesonderte Herstellung eines Säurehalogenids und die zusätzliche Verwendung großer Mengen von Phosphoroxychlorid werden vermieden und somit Anlagen, Regel-, Steuer- und Kontrolleinrichtungen eingespart. Da es im großtechnischen Maßstab im Hinblick auf Betriebssicherheit, Toxizität der Komponenten, Maßnahmen zum Schutz des Betriebspersonals bzw. Abwasser- und Abgasfragen nur mit besonderen Maßnahmen möglich ist, große Mengen an diesen Suspensionen von Phosphorpentachlorid zu handhaben, ist das erfindungsgemäße Verfahren einfacher, wirtschaftlicher, betriebssicherer, weniger toxisch und umweltfreundlicher. Alle diese vorteilhaften Ergebnisse des erfindungsgemäßen Verfahrens sind im Hinblick auf den Stand der Technik überraschend. Insbesondere hätte man einen direkten Angriff von Halogen auf den Ausgangsstoff bzw. eine verringerte Bildung von Phosphorhalogeniden und somit eine Blockierung der Reaktion oder erheblich verringerte Herstellung von Sulfamidsäurehalogeniden erwarten sollen. So ist es z. B. aus Houben—Weyl, Methoden der Organischen Chemie, Band 5/3, Seiten 796 bis 798, und Ullmanns Encyklopädie der technischen Chemie (3. Auflage), Band 5, Seiten 384 und 385, bekannt, daß Ammoniak, primäre und sekundäre Amine sowie Carbamidsäureester mit Chlor zu den entsprechenden N-Monochlor- bzw. N,N-Dichlorverbindungen umgesetzt werden; die Umsetzung erfolgt sowohl im alkalischen wie nicht alkalischen Bereich. Ebenfalls zeigt die deutsche Auslegeschrift 10 01 254 die Chlorierung von Stickstoffverbindungen wie Biguanidderivaten, in saurer Lösung.

Bevorzugte Ausgangsstoffe II, III, IV und dementsprechend bevorzugte Endstoffe I sind solche, in deren Formeln R einen geradkettigen oder verzweigten Alkylrest mit 1 bis 20, insbesondere 1 bis 8

Kohlenstoffatomen, durch mehrere Alkoxygruppen, vorzugsweise 3 oder 2 Alkoxygruppen und insbesondere eine Alkoxygruppe mit 1 bis 7, insbesondere 1 bis 3 Kohlenstoffatomen substituierten Alkylrest mit 2 bis 20, insbesondere 2 bis 8, vorteilhaft 2 bis 6 Kohlenstoffatomen, oder einen Cycloalkylrest mit 4 bis 8 Kohlenstoffatomen bedeutet, und X ein Bromatom oder insbesondere Chloratom bezeichnet. Die genannten Reste können noch durch unter den Reaktionsbedingungen inerte Gruppen und/oder Atome, z. B. Chloratome, Bromatome, Alkylgruppen, Alkoxygruppen mit jeweils 1 bis 4 Kohlenstoffatomen, Carbalkoxygruppen mit 2 bis 4 Kohlenstoffatomen, Cycloalkylgruppen mit 4 bis 6 Kohlenstoffatomen, substituiert sein.

Beispielsweise kommen folgende Sulfamidsäuren II in Betracht:

Methylsulfamidsäure, Äthylsulfamidsäure, n-Propylsulfamidsäure, Isopropylsulfamidsäure, n-Butylsulfamidsäure, Isobutylsulfamidsäure, sek.-Butylsulfamidsäure, Cyclobutylsulfamidsäure, 1-Äthyl-1-propylsulfamidsäure, 1,2-Dimethyl-1-propylsulfamidsäure, n-Pentylsulfamidsäure, Cyclopentylsulfamidsäure, n-Hexylsulfamidsäure, Hexyl-(3)-sulfamidsäure, Cyclohexylsulfamidsäure, Cycloheptylsulfamidsäure, Heptyl-(4)-sulfamidsäure, Cyclooctylsulfamidsäure, 2-Methyl-1-äthyl-1-propylsulfamidsäure, 1, 2, 2-Trimethyl-1-propylsulfamidsäure, 1,3-Dimethyl-1-n-butylsulfamidsäure, 1,2-Dimethyl-1-n-butylsulfamidsäure, 1,2-Dimethyl-1-n-hexylsulfamidsäure, 1-Cyclohexyl-1-äthylsulfamidsäure, 2-Chlor-isopropylsulfamidsäure, 2-Chlorpropylsulfamidsäure, 3-Chlor-propylsulfamidsäure, 3-Brompropylsulfamidsäure, 1-Chlormethyl-1-propylsulfamidsäure tert.-Butyl-, Pentyl-(2)-, n-Heptyl-, n-Octyl-, n-Nonyl-, n-Decyl-, 2-Äthylhexyl-, 2-Äthylpentyl-, 3-Äthylpentyl-, 2,3-Dimethyl-n-butyl-, 2-Methylpentyl-, 3-Methylpentyl-, 2-Methylheptyl- 3-Methylheptyl-, 4-Methylheptyl-, 3-Äthylhexyl-, 2,3-Dimethylhexyl-, 2,4-Dimethylhexyl-, 2,5-Dimethylhexyl-, Undecyl-, Dodecyl-, Tridecyl-, Tetradecyl-, Pentadecyl-, Hexadecyl-, Heptadecyl-, Octadecyl-, Nonadecyl-, Eicosyl-sulfamidsäure; die ω-Methoxy-, ω-Äthoxy-, ω-n-Propoxy-, ω-Isopropoxy-, ω-n-Butoxy-, ω-Isobutoxy-, ω-sek.-Butoxy-, ω-tert.-Butoxy-, ω-Pentoxy-, ω-Pentoxy-(2)-, ω-Pentoxy-(3)-, ω-n-Hexoxy-, ω-n-Heptoxy-verbindung der Äthyl-, n-Propyl-, Isopropyl-, n-Butyl-, Isobutyl-, sek.-Butyl-, tert.-Butyl-, Pentyl-, Pentyl-(2)-, Pentyl-(3)-, n-Hexyl-, n-Heptyl-, n-Octyl-, n-Nonyl-, n-Decyl-, 2-Äthylhexyl-, 2-Äthylpentyl-, 3-Äthylpentyl-, 2,3-Dimethyl-n-butyl-, 2-Methylpentyl-, 3-Methylpentyl-, 2-Methylheptyl-, 3-Methylheptyl-, 4-Methylheptyl-, 3-Äthylhexyl-, 2,3-Dimethylhexyl-, 2,4-Dimethylhexyl-, 2,5-Dimethylhexyl-, Undecyl-, Dodecyl-, Tridecyl- Tetradecyl-, Pentadecyl-, Hexadecyl-, Heptadecyl-, Octadecyl-, Nonadecyl-, Eicosyl-sulfamidsäure; entsprechende Methyl-, Äthyl-, n-Propyl-, Isopropyl-, n-Butyl-, Isobutyl-, sek.-Butyl-, tert.-Butyl-, Pentyl-, Pentyl-(2)-, Pentyl-(3)-, n-Hexyl-, n-Heptyl-äther in 1-Stellung oder 2-Stellung der n-Propyl-, Isopropyl-, n-Butyl-, Isobutyl-, sek.-Butyl-, tert.-Butyl-, Pentyl-, Pentyl-(2)-, Pentyl-(3)-, n-Hexyl-, n-Heptyl-, n-Octyl-, n-Nonyl-, n-Decyl-, 2-Äthylhexyl-, 2-Äthylpentyl-, 3-Äthylpentyl-, 2,3-Dimethyl-n-butyl-, 2-Methylpentyl-, 3-Methylpentyl-, 2-Methylheptyl-, 3-Methylheptyl-, 4-Methylheptyl-, 3-Äthylhexyl-, 2,3-Dimethylhexyl-, 2,4-Dimethylhexyl-, 2,5-Dimethylhexyl-, Undecyl-, Dodecyl-, Tridecyl-, Tetradecyl-, Pentadecyl-, Hexadecyl-, Octadecyl-, Nonadecyl-, Eicosyl-sulfamidsäure bzw. 1-Stellung der Äthylsulfamidsäure.

Die Ausgangsstoffe II können in Gestalt von Sulfamidsäuren, z. B. nach den in den deutschen Offenlegungsschriften 24 24 371 und 21 64 197 beschriebenen Verfahren hergestellten, schwefelsäurefreien Sulfamidsäuren, oder ihrer sulfamidsauren Metallsalze angewendet werden. Bevorzugte Metallsalze sind Alkali- oder Erdalkalisalze wie sulfamidsaures Magnesium, Calcium, Lithium, Kalium und insbesondere Natrium. Man kann schwarzen, roten und vorzugsweise gelben Phosphor verwenden. Als Halogen sind Brom und insbesondere Chlor, als Phosphortrihalogenid Phosphortribromid und insbesondere Phosphortrichlorid bevorzugt. Halogen, Phosphor und/oder Phosphortrichlorid können jeweils in stöchiometrischer Menge oder im Überschuß mit Ausgangsstoff II umgesetzt werden, vorteilhaft in einer Menge von 1 bis 1,5, vorzugsweise von 1 bis 1,2 Mol Phosphortrihalogenid oder Grammatom Phosphor, bezogen auf ein Mol Ausgangsstoff II, und/oder vorteilhaft 1 bis 1,5, insbesondere 1 bis 1,2 Mol Halogen bei Verwendung von $PCl_3$ oder vorteilhaft 2,5 bis 3,3, insbesondere 2,5 bis 3 Mol Halogen bei Verwendung von Phosphor, bezogen auf ein Mol Ausgangsstoff II.

Die Reaktion wird in der Regel bei einer Temperatur von —10 bis 130° C, vorzugsweise 10 bis 120° C, insbesondere von 40 bis 100° C, drucklos oder unter Druck, kontinuierlich oder diskontinuierlich durchgeführt. Man verwendet unter den Reaktionsbedingungen inerte organische Lösungsmittel, wobei vorteilhaft im Falle der Verfahrensweisen b) und c) die Gesamtmenge an organischem Lösungsmittel schon dem ersten Reaktionsschritt zugegeben wird. Als Lösungsmittel kommen z. B. in

sulfamidsaures Metallsalz mit Halogen als Halogenierungsmittel in Gegenwart von Phosphor oder Phosphortrihalogenid umsetzt, oder

c) einen substituierten Harnstoff der Formel

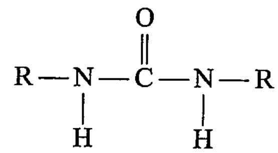

(IV)

worin R die vorgenannte Bedeutung hat, mit Oleum, das Schwefeltrioxid in einer Menge von 1 bis 2,5 Mol und Schwefelsäure in einer Menge von 1 bis 1,5 Mol je Mol Ausgangsstoff II enthält, in einem ersten Schritt bei einer Temperatur von −20 bis +50° C und dann in einem zweiten Schritt bei einer Temperatur zwischen 50° C und 140° C umsetzt, und dann die erhaltene Sulfamidsäure der Formel

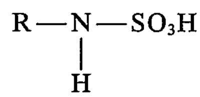

(II)

in der R die vorgenannte Bedeutung hat, oder ihr sulfamidsaures Metallsalz in einem dritten Schritt mit Halogen als Halogenierungsmittel in Gegenwart von Phosphor oder Phosphortrihalogenid umsetzt.

**Claim**

A process for the preparation of a sulfamyl halide of the formula

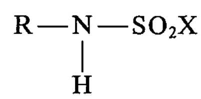

(I)

where R is an aliphatic or cycloaliphatic radical and X is halogen, by reacting a sulfamic acid or a metal salt there of with a halogenating agent in the presence of a solvent, characterized in that

a) a sulfamic acid of the formula

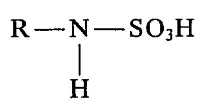

(II)

where R has the above meaning, or a metal salt thereof, is reacted with a halogen as the halogenating agent in the presence of phosphorus or a phosphorus trihalide, or

b) in a first stage, an isocyanate of the formula

$$R-N=C=O \qquad \text{(III)}$$

where R has the above meaning, ist reacted with sulfuric acid to give a sulfamic acid of the formula

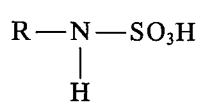

(II)

where R has the above meaning, and thereafter, in a second stage, compound II or a metal salt thereof is reacted with a halogen as the halogenating agent, in the presence of phosphorus or a phosphorus trihalide, or

c) a substituted urea of the formula

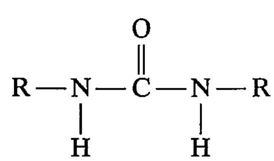

(IV)

where R has the above meaning, is reacted with oleum, which contains from 1 to 2.5 moles of sulfur trioxide and from 1 to 1.5 moles of sulfuric acid per mole of starting material II, in a first step at from −20 to +50° C an then in a second step at from 50° C to 140° C, after which the resulting sulfamic acid of the formula

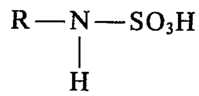 (II)

where R has the above meaning, or a metal salt thereof, is reacted, in a third step, with a halogen as the halogenating agent, in the presence of phosphorus or a phosphorus trihalide.

**Revendication**

Procédé de préparation d'halogénures d'acides sulfamiques de la formule

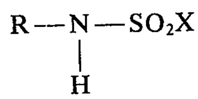 (I)

dans laquelle R désigne un reste aliphatique ou cycloaliphatique et X un atome d'halogène, par réaction d' acides sulfamiques ou de sels de métaux d'acides sulfamiques avec un agent d'halogénation en présence d'un solvant, caractérisé en ce que:

a) on fait réagir un acide sulfamique de la formule

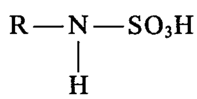 (II)

dans laquelle R a la signification définie, ou un sel de métal d'un tel acide sulfamique, avec un halogène en tant qu'agent d'halogénation en présence de phosphore ou d'un trihalogénure de phosphore, ou

b) on fait réagir dans un premier stade un isocyanate de la formule

$$R—N=C=O \qquad (III)$$

dans laquelle R a la signification définie, avec l' acide sulfurique et l'acide sulfamique abtenu, de la formule

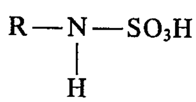 (II)

dans laquelle R a la signification définie, ou un sel de métal du composé II, dans un deuxième stade avec un halogène en tant qu'agent d'halogénation en présence de phosphore ou d'un trihalogénure de phosphore, ou

c) on fait réagi une urée substituée de la formule

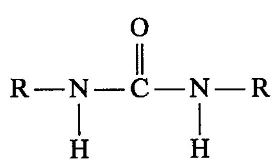 (IV)

dans laquelle R a la signification définie, avec de l'oléum avec une teneur en anhydride sulfurique de 1 à 2,5 moles et en acide sulfurique de 1 à 1,5 mole par mole de composé de départ II, dans un premier stade à une temérature entre −20 et +50° C, puis dans un deuxième stade entre 50 et 140° C, et ensuite l'acide sulfamique abtenu, de la formule

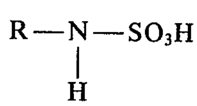 (II)

dans laquelle R a la signification définie, ou un sel de métal de cet acide, dans un troisième stade avec un halogène en tant qu'agent d'halogénation en présence de phosphore ou d'un trihalogénure de phosphore.